(19)

Europäisches
Patentamt
European
Patent Office
Office européen
des brevets

(11) **EP 4 393 481 A1**

(12) **EUROPEAN PATENT APPLICATION**
published in accordance with Art. 153(4) EPC

(43) Date of publication:
**03.07.2024 Bulletin 2024/27**

(21) Application number: **22885750.4**

(22) Date of filing: **19.10.2022**

(51) International Patent Classification (IPC):
**A61K 9/12** (2006.01)    **A61K 31/475** (2006.01)
**A61M 11/00** (2006.01)

(52) Cooperative Patent Classification (CPC):
**A61K 9/0073; A61K 9/12; A61K 31/4375;
A61K 31/475; A61K 36/74; A61K 47/10;
A61K 47/12; A61M 11/00; A61M 11/02;
A61M 11/04; A61P 13/02; A61P 13/08; A61P 15/10**

(86) International application number:
**PCT/CN2022/126035**

(87) International publication number:
**WO 2023/071872 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.10.2021 CN 202111248947**

(71) Applicant: **Shenzhen First Union Technology Co.,
Ltd.
Shenzhen, Guangdong 518000 (CN)**

(72) Inventors:
• **DENG, Jingjing
  Shenzhen, Guangdong 518000 (CN)**
• **XU, Zhongli
  Shenzhen, Guangdong 518000 (CN)**
• **LI, Yonghai
  Shenzhen, Guangdong 518000 (CN)**

(74) Representative: **Jacob, Reuben Ellis et al
Maucher Jenkins
Seventh Floor Offices
Artillery House
11-19 Artillery Row
London SW1P 1RT (GB)**

(54) **LIQUID PREPARATION AND PREPARATION METHOD THEREFOR, AEROSOL GENERATION
SYSTEM, CARTRIDGE FOR AEROSOL GENERATION SYSTEM, AND AEROSOL GENERATION
APPARATUS**

(57)    The present application discloses a liquid preparation, a method for preparing the liquid preparation, an aerosol generation system, a cartridge for the aerosol generation system, and an aerosol generation apparatus. The liquid preparation includes a yohimbine extract, a chaotropic agent and a solvent, where the solubility of the yohimbine extract in the chaotropic agent is greater than the solubility of the yohimbine extract in the solvent; and the yohimbine extract can form a stable and transparent solution with the solvent after being dissolved in the chaotropic agent. The yohimbine extract is dissolved in the chaotropic agent and then mixed with the solvent in the liquid preparation, the use amount of the yohimbine extract in the aerosol generation system is increased by means of dissolving the yohimbine extract in the chaotropic agent, then mixing with the solvent of the liquid preparation, thereby promoting the use of the yohimbine extract in the aerosol generation system.

EP 4 393 481 A1

**Description**

**CROSS-REFERENCE TO RELATED APPLICATIONS**

The present application claims priority to Chinese Patent Application No. 202111248947.0, filed with the China National Intellectual Property Administration on October 26, 2021 and entitled "LIQUID PREPARATION AND PREPARATION METHOD THEREFOR, AEROSOL GENERATION SYSTEM, CARTRIDGE FOR AEROSOL GENERATION SYSTEM, AND AEROSOL GENERATION APPARATUS", which is incorporated herein by reference in its entirety.

**TECHNICAL FIELD**

The present application relates to the technical field of atomization, and particularly relates to a liquid preparation containing yohimbine, a method for preparing the liquid preparation, an aerosol generation system, a cartridge for the aerosol generation system, and an aerosol generation apparatus.

**BACKGROUND**

An aerosol generation system includes an aerosol generation apparatus and a liquid preparation stored in the aerosol generation apparatus. The aerosol generation apparatus atomizes the liquid preparation to form an aerosol. The liquid preparation mainly includes glycerol, 1,2-propylene glycol, synthetic essence, nicotine and/or nicotine salt, and the like. In addition to serving as a substitute for traditional combustion type cigarette products, the aerosol generation system can also be used for adjuvant therapy. The main method is to add health-care functional components to liquid preparations, and the health-care functional components are atomized to form aerosols for users to inhale. In recent years, the big health industry has developed rapidly, and liquid preparations with health-care functional components have attracted increasing attention from researchers and consumers. The focus of attention is to select health-care functional components that are truly beneficial to users and add the health-care functional components to liquid preparations, so as to play roles of the health-care functional components.

The yohimbine tree is a herbaceous plant with a hundred year history in Africa, and there are also many health-care products that use a yohimbine extract as raw material on the market. In the prior art, the yohimbine extract often exists in the form of yohimbine hydrochloride, but the yohimbine hydrochloride has low solubility in liquid preparations of a glycerol-propylene glycol system and is extremely easy to precipitate and form turbid liquid. As a result, yohimbine is difficult to be converted to aerosols for users to inhale, thus greatly hindering the use of the yohimbine extract in the aerosol generation system.

**SUMMARY**

In order to solve the problem of difficult conversion of a yohimbine component in a liquid preparation containing yohimbine in the prior art, an example of the present application provides a liquid preparation for an aerosol generation system. The liquid preparation includes a yohimbine extract, a chaotropic agent and a solvent, where the solubility of the yohimbine extract in the chaotropic agent is greater than the solubility of the yohimbine extract in the solvent; and the yohimbine extract can form a stable and transparent solution with the solvent after being dissolved in the chaotropic agent.

In some examples, based on the mass percentage of the liquid preparation, the mass percentage content of the yohimbine extract is 0.01-15%; the mass percentage content of the chaotropic agent is 0.09-15%; and the mass percentage content of the solvent is 50-99.9%.

In some examples, the yohimbine extract includes yohimbine.

In some examples, the yohimbine and the chaotropic agent form a yohimbine concentrated solution; and based on the weight percentage of the liquid preparation, the mass percentage content of the yohimbine concentrated solution is 0.1-30%.

In some examples, the solvent includes glycerol and propylene glycol.

In some examples, based on the weight percentage of the liquid preparation, the mass percentage content of the yohimbine extract is 0.015-12%; or, the mass percentage content of the yohimbine extract is 0.02-12%.

In some examples, the chaotropic agent includes at least one of benzyl alcohol and organic acid.

In some examples, the chaotropic agent further includes water.

In some examples, the organic acid includes at least one of lactic acid, glacial acetic acid, adipic acid, fumaric acid, tartaric acid, malic acid, citric acid and benzoic acid.

In some examples, based on the weight percentage of the liquid preparation, the mass percentage content of the benzyl alcohol is 0-27%; or, the mass percentage content of the benzyl alcohol is 0.008-15%; or, the mass percentage content of the benzyl alcohol is 0.012-9%.

In some examples, based on the weight percentage of the liquid preparation, the mass percentage content of the organic acid is 0-24%; or, the mass percentage content of the organic acid is 0.008-15%; or, the mass percentage content of the organic acid is 0.012-9%.

In some examples, based on the weight percentage of the liquid preparation, the mass percentage content of the water is 0-24%; or, the mass percentage content of the water is 0.01-15%; or, the mass percentage content of the water is 0.02-12%.

In some examples, the liquid preparation further includes at least one of a sweetening agent, a cooling agent and a flavor composition.

An example of the present application further provides a method for preparing the foregoing liquid preparation, including the following steps: preparing yohimbine; adding the yohimbine to a chaotropic agent to enable the yohimbine to be fully dissolved in the chaotropic agent to obtain a yohimbine concentrated solution; and adding the yohimbine concentrated solution to a solvent.

In some examples, the yohimbine is prepared from yohimbine hydrochloride by an acidification process.

The present application further provides a cartridge for an aerosol generation system. The cartridge contains the foregoing liquid preparation.

The present application further provides an aerosol generation system, including the foregoing cartridge and an aerosol generation apparatus. The aerosol generation apparatus is constructed to receive at least a portion of the cartridge and atomize the liquid preparation to form an aerosol.

An example of the present application further provides an aerosol generation apparatus, including an atomizer and a chamber containing the foregoing liquid preparation. The atomizer is configured to atomize the liquid preparation to generate an aerosol.

The examples of the present application have the following beneficial effects: in the foregoing liquid preparation, by dissolving the yohimbine extract in the chaotropic agent, because the solubility of the yohimbine extract in the chaotropic agent is greater than the solubility of the yohimbine extract in the solvent, the yohimbine extract can form a stable and transparent solution with the solvent in the liquid preparation after being dissolved in the chaotropic agent, the use amount of the yohimbine extract in the aerosol generation system is increased, thereby promoting the use of the yohimbine extract in the aerosol generation system.

## BRIEF DESCRIPTION OF THE DRAWINGS

One or more examples are exemplarily described with reference to the figures in the corresponding accompanying drawings, and the exemplary descriptions do not constitute limitations to the examples. Unless otherwise particularly stated, the figures in the accompanying drawings are not drawn to scale.

FIG. 1 is HPLG spectrograms of yohimbine hydrochloride (a) and yohimbine sodium carbonate (b) before and after a deacidification process provided in an example of the present application;

FIG. 2 is HPLG spectrograms of aerosols formed by atomization of yohimbine hydrochloride and yohimbine sodium carbonate provided in an example of the present application;

FIG. 3 is a comparison diagram of dissolution situations of three groups of solutions, namely a liquid preparation a, a liquid preparation b and a liquid preparation c, provided in an example of the present application; and

FIG. 4 is a comparison diagram of conversion rates of four groups of yohimbine provided in an example 11 of the present application.

## DETAILED DESCRIPTION

For ease of understanding of the present application, the present application is described below in more detail with reference to the accompanying drawings and specific implementations.

The yohimbine extract is an active substance extracted from the bark of a natural plant "YOHINME" growing in Africa. The yohimbine can act on the sexual organ to expand the blood vessels of the sexual organ, increase the blood flow volume of the sexual organ and increase the formation of nitrous oxide, thereby enhancing the sexual function. The yohimbine is currently one of the most recognized and widely used plant-based pure plant preparations for treating the erectile dysfunction, and can prevent prostatic hyperplasia and urinary system infections. The yohimbine extract is added to a liquid preparation used by an aerosol generation system, an aerosol generation apparatus is atomized to form an aerosol, and then, a user can inhale the aerosol, so that the yohimbine can exert functions and therapeutic effects. Therefore, applying the yohimbine to the field of the aerosol generation system can greatly improve the healthy use effects of the aerosol generation apparatus.

The yohimbine extract includes yohimbine hydrochloride and yohimbine. The chemical formula of the yohimbine is C21H26N203. The yohimbine is easily soluble in chloroform, soluble in methanol and ethanol, and slightly soluble in water. The commercially available yohimbine extract mainly exists in the form of yohimbine hydrochloride. The chemical formula of the yohimbine hydrochloride is C21H27CLN203. The yohimbine hydrochloride is easily soluble in chloroform, soluble in methanol and ethanol, and slightly soluble in water. Through experiments, it is found that the yohimbine hydrochloride and the yohimbine both have extremely low solubility in a solvent of a glycerol-propylene glycol system. Calculated by the weight percentage of the liquid preparation, when the addition amounts of the yohimbine hydrochloride and the yohimbine are much lower than 1%, crystals are precipitated. Because the yohimbine extract is insoluble in the solvent, the yohimbine is difficult to be converted to aerosols for the users to inhale.

In order to promote the effective use of the yohimbine extract in the aerosol generation system, Example 1 of the present application provides a liquid preparation containing the yohimbine extract for the aerosol generation system. The liquid preparation includes a solvent and a yohimbine concentrated solution. The yohimbine extract exists in the yohimbine concentrated solution. The yohimbine concentrated solution can form a stable and transparent solution with a solvent of a glycerol-propylene glycol system, thereby solving the problem of difficult dissolution of the yohimbine extract in the solvent of the glycerol-propylene glycol system.

Based on the weight percentage of the liquid preparation, the mass percentage content of the yohimbine concentrated solution is 0.1-30%; and the mass percentage content of the solvent is 50-99.9%. The yohimbine concentrated solution includes the yohimbine extract. Based on the weight of the liquid preparation, the mass percentage content of the yohimbine extract is 0.01-15%; or the mass percentage content of the yohimbine extract is 0.015-12%; or the mass percentage content of the yohimbine extract is 0.02-12%.

Further, in the yohimbine concentrated solution, based on the weight percentage of the yohimbine concentrated solution, the mass percentage of the yohimbine extract is 10-50%; optionally, the mass percentage of the yohimbine extract is 15-40%; and further, the mass percentage of the yohimbine extract may also be 20-40%. Based on the above, based on the weight percentage of the liquid preparation, the mass percentage of the yohimbine extract is 0.01-15%; optionally, the mass percentage of the yohimbine extract is 0.015-12%; and further, the mass percentage of the yohimbine extract may be 0.02-12%. Optionally, the specific addition amount of the yohimbine extract may be adjusted according to the needs of users. For example, based on the weight percentage of the liquid preparation, the mass percentage of the yohimbine extract may be 1%, 2%, 5%, 6%, 8%, 10%, 12%, 14%, or any value between 0.01% and 15%. Because a large dose of the yohimbine extract can be dissolved in the chaotropic agent to form a yohimbine concentrated solution, the addition amount of the yohimbine extract in the liquid preparation can be arbitrarily changed by adjusting the proportion of the yohimbine concentrated solution in the liquid preparation.

Example 1 of the present application further provides a portion of specific component formulas for preparing the foregoing liquid preparation. Based on the total weight percentage of the liquid preparation, the mass percentage content of each component is shown in Table 1 below.

Table 1 Formulas of liquid preparation

|   | Yohimbine concentrated solution | Glycerol | Propylene glycol |
|---|---|---|---|
| 1 | 30% | 50% | 20% |
| 2 | 20% | 60% | 20% |
| 3 | 10% | 80% | 10% |
| 4 | 1% | 70% | 29% |
| 5 | 0.1% | 70%/ | 29.9% |

The yohimbine extract mainly exists in the form of yohimbine in the yohimbine concentrated solution. In some embodiments, the solvent mainly includes glycerol and propylene glycol, and the glycerol and the propylene glycol may be mixed in any ratio. In an optional embodiment, the solvent may further include one or several of butanediol, 1,2,4-butantriol, dipropylene glycol, diethylene glycol, triethylene glycol, polyethylene glycol 200, polyethylene glycol 400, glycerol triacetate, dipropylene glycol ether, ethanol, water, triethyl citrate and caprylic/capric triglyceride. The specific solvent needs to be optimized and selected according to the types of components added to the solvent. From the perspective of improving the mass of the aerosol generated by the aerosol generation apparatus, the glycerol and the propylene glycol are preferred components in the solvent.

Because the source of yohimbine is limited, in Example 2 provided in the present application, the yohimbine is prepared from yohimbine hydrochloride by a deacidification process. The deacidification process of an organic substance may be performed in a manner of oxidation or acid-base neutralization, where the manner of acid-base neutralization includes an esterification reaction. The esterification reaction is taken as an example for specific description. The deacidification process of the yohimbine hydrochloride mainly includes the following steps:

Step 1: Esterification reaction

Yohimbine hydrochloride powder was added to ethyl acetate; and after the yohimbine hydrochloride powder was fully dissolved, the solution was transferred to a separating funnel, purified water was added, the separating funnel was fully shaken for mixing uniformly, and then, the solution stood for layering. Calculated by the weight ratio, the ratio of the yohimbine hydrochloride to the ethyl acetate was 1:(40-70); and calculated by the volume ratio, the ratio of the purified water to the ethyl acetate was 1 :(2-3).

Step 2: PH value adjustment and filtration

After the solution was layered, a water layer and an ethyl acetate layer were formed, where the upper layer was the ethyl acetate layer, and the lower layer was the water layer; a sufficient sodium hydroxide solution was added, and the separating funnel was fully shaken until the PH value of the mixed solution was about 9; and after the solution stood for layering, the water at the lower layer was released.
Then, enough purified water was added for washing the upper ethyl acetate layer for 3 times. The concentration of the sodium hydroxide solution was 2-6 mol/L; and calculated by the volume ratio, the ratio of the purified water to the ethyl acetate was 1 :(2-3).

Step 3: Purification of yohimbine

The ethyl acetate layer in the foregoing step was collected, the mixed solution was placed in a rotary evaporator for rotary evaporation under reduced pressure until the liquid in a rotary bottle was converted to dry solid, and the foregoing powdered solid was taken out to obtain the yohimbine.
Step 3 was repeated for repeated purification to prepare yohimbine powder with a purity higher than 98% (based on the mass percentage).
In Example 3 provided in the present application, by detection and comparative analysis, whether the deacidification process in Example 2 affects the functional components of the yohimbine was verified. The detection and analysis method is as follows: the yohimbine hydrochloride before the deacidification process and the yohimbine after the deacidification process were respectively added to a liquid chromatograph, and then, whether the deacidification process affects the functional components of the yohimbine was verified by comparative analysis of the liquid chromatograph. The manner of comparative analysis of the liquid chromatograph is specifically described below:

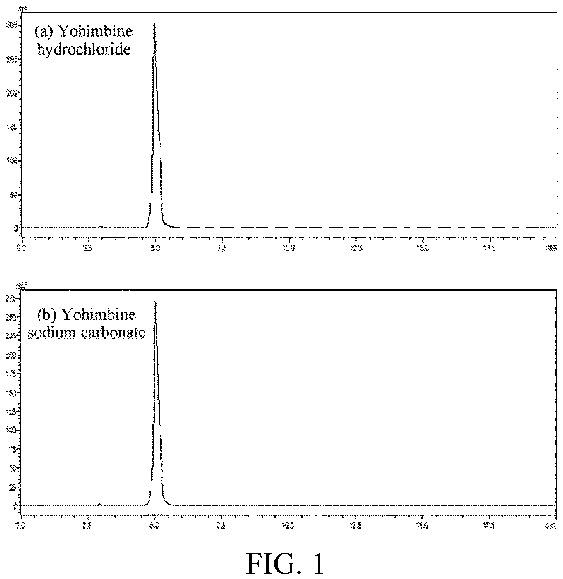

(1) Chromatographic conditions: DGU-20A5R type liquid chromatograph, reversed phase chromatography column C18, 4.6x250 mm, 3.5 um; mobile phase: acetonitrile-water (volume ratio: 30:70); column temperature: 35°C, flow rate: 1.0 mL/min; and detection wavelength: 278 nm, sample injection: 10 μL.
(2) The yohimbine hydrochloride and the yohimbine obtained by the deacidification process were added to the chromatograph for liquid chromatography analysis. By the comparative analysis of the liquid chromatograph, the obtained chromatograms are shown in FIG. 1, and the peak ranges of the two chromatograms remain consistent. The foregoing chromatography analysis indicates that the deacidification process provided in Example 2 of the present application does not affect the active functional groups of the yohimbine.

In Example 4 provided in the present application, further, the yohimbine hydrochloride before the deacidification process was prepared into an atomized liquid preparation 1, and the yohimbine after the deacidification process was prepared into an atomized liquid preparation 2; the liquid preparation 1 was added to the aerosol generation apparatus for atomization to obtain an aerosol 1, and the liquid preparation 2 was added to the aerosol generation apparatus for atomization to obtain an aerosol 2; and the liquid chromatograph was used for performing comparative analysis of the properties of the aerosol 1 and the aerosol 2. The specific manner of comparative analysis of the liquid chromatograph was the same as that of the foregoing solution 1 and solution 2, referring to the following description:

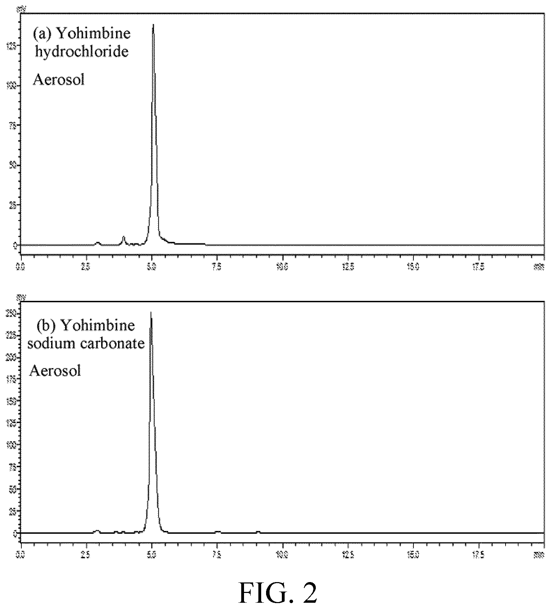

(1) Chromatographic conditions: DGU-20A5R type liquid chromatograph, reversed phase chromatography column C18, 4.6x250 mm, 3.5 um; mobile phase: acetonitrile-water (volume ratio: 30:70); column temperature: 35°C, flow rate: 1.0 mL/min; and detection wavelength: 278 nm, sample injection: 10 μL.
(2) The aerosol 1 and the aerosol 2 were respectively sampled and added to the liquid chromatograph for liquid chromatography analysis. By the comparative analysis of the liquid chromatograph, the obtained chromatograms are shown in FIG. 2, the peak ranges of the two chromatograms basically remain consistent, and the peak ranges in FIG. 2 and FIG. 1 are also basically consistent. The foregoing chromatography analysis indicates that the aerosol 1 contains the yohimbine component, and the aerosol 2 contains the yohimbine component. Both the yohimbine hydrochloride and the yohimbine can be absorbed by the human body in the form of aerosols, thereby further achieving health care effects. It should be noted that, both the liquid preparation 1 and the liquid preparation 2 do not use solvents of a glycerol-propylene glycol system, so both the yohimbine hydrochloride and the yohimbine can be effectively converted to aerosols.

In order to increase the solubility of the yohimbine in the solvent of the propylene glycol-glycerol system to the maximum extent, the yohimbine obtained by the foregoing deacidification process is prepared into a yohimbine concentrated solution. Example 5 of the present application provides formulas of the yohimbine concentrated solution. The yohimbine concentrated solution includes yohimbine and a chaotropic agent, where the yohimbine can be fully dissolved in the chaotropic agent to form a stable and transparent solution, and precipitates of the yohimbine cannot be generated. The chaotropic agent includes benzyl alcohol, organic acid and water, where the benzyl alcohol and the organic acid serve as preferred components of the chaotropic agent. The organic acid includes, but is not limited to, one or a mixture of several of lactic acid, glacial acetic acid, adipic acid, fumaric acid, malic acid, citric acid and benzoic acid. The organic acid is preferably the lactic acid and the glacial acetic acid. Regarding the dissolution mechanism of the yohimbine in the chaotropic agent, on the one hand, the yohimbine can form intermolecular hydrogen bonds with the benzyl alcohol or the organic acid, thereby promoting the dissolution of the yohimbine in benzyl alcohol or organic acid solvents. On the other hand, the yohimbine is a polar organic substance, and the benzyl alcohol, the glacial acetic acid, the lactic acid and the like are polar organic solvents, which can promote the dissolution of phenylyohimbine in the foregoing two substances. Because the polarity of the propylene glycol and the glycerol is lower than the polarity of the benzyl alcohol, the glacial acetic acid and the water, according to the principle of like dissolves like, polar yohimbine is more easily soluble in the chaotropic agent with a relatively high polarity formed by mixing the benzyl alcohol, the glacial acetic acid, the lactic acid and the water.

Based on the weight percentage of the yohimbine concentrated solution, the mass percentage of the yohimbine is 10-50%; optionally, the mass percentage of the yohimbine is 15-40%; and further, the mass percentage of the yohimbine may also be 20-40%. In the yohimbine concentrated solution, based on the weight percentage of the yohimbine concentrated solution, the mass percentage of the benzyl alcohol is 0-90%; optionally, the mass percentage of the benzyl alcohol is 8-50%; and further, the mass percentage of the benzyl alcohol may also be 12-30%. In the yohimbine concentrated solution, based on the weight percentage of the yohimbine concentrated solution, the mass percentage of the organic acid is 0-80%; optionally, the mass percentage of the organic acid is 8-50%; and further, the mass percentage of the organic acid may also be 12-30%. In the yohimbine concentrated solution, based on the weight percentage of the yohimbine concentrated solution, the mass percentage of the water is 0-80%; optionally, the mass percentage of the water is 10-50%; and further, the mass percentage of the water may also be 20-40%. It can be understood that the chaotropic agent can be selected from any organic substance that can promote the dissolution of the yohimbine or the yohimbine hydrochloride. However, the chaotropic agent needs to meet the atomization function requirements of the atomized liquid preparation of the aerosol generation system, and can form an aerosol suitable for suction without affecting the properties of the aerosol formed by atomization of the liquid preparation, where the properties include taste, smoke stability, user health, and the like.

In the liquid preparation, based on the weight percentage of the liquid preparation, the mass percentage of the benzyl alcohol is 0-27%; optionally, the mass percentage of the benzyl alcohol is 0.008-15%; and optionally, the mass percentage content of the benzyl alcohol is 0.012-9%. It can be understood that the specific addition dose of the benzyl alcohol can be adaptively adjusted according to the addition amount need of the yohimbine that needs to be dissolved, and the mass ratio content of the benzyl alcohol may be any value between 0% and 27%.

In the liquid preparation, based on the weight percentage of the liquid preparation, the mass percentage content of the organic acid is 0-24%; optionally, the mass percentage content of the organic acid is 0.008-15%; and optionally, the mass percentage content of the organic acid is 0.012-9%. It can be understood that the specific addition dose of the organic acid can be adaptively adjusted according to the addition amount need of the yohimbine that needs to be dissolved, and the mass ratio content of the organic acid may be any value between 0 and 24%.

The specific content of the chaotropic agent in the yohimbine concentrated solution can be adaptively adjusted according to the content need of the yohimbine extract that needs to be added, and is not limited to the foregoing content range. For example, if the content need of the yohimbine extract in the liquid preparation for the aerosol generation system is 3% (based on the mass percentage), in the liquid preparation, the content of the yohimbine concentrated solution is 15% (based on the mass percentage), thus in the yohimbine concentrated solution, the content of the yohimbine extract is 20% (based on the mass percentage), and the content of the chaotropic agent is 80% (based on the mass percentage).

Example 6 of the present application further provides a process for preparing the foregoing yohimbine concentrated solution, including the following steps:

1. yohimbine powder was weighed, and a benzyl alcohol solvent was added to enable the benzyl alcohol to fully infiltrate the yohimbine powder;

2. purified water was added to further infiltrate the yohimbine powder in the foregoing solution;

3. glacial acetic acid was added to the foregoing solution;

4. a container containing the foregoing solution was sealed, and the solution was heated and stirred at 60°C or eddied to enable the yohimbine powder in the solution to be fully dissolved; and

5. impurities in the foregoing solution were filtered to obtain the yohimbine concentrated solution.

It should be noted that, in the process of preparing the yohimbine concentrated solution, the content of each component is different, the corresponding dissolution situation of the yohimbine powder is different, the purity of the used yohimbine is greater than 98%, and there are still a small amount of impurities. In yohimbine concentrated solutions prepared according to some formulas, the yohimbine can be completely dissolved. In yohimbine concentrated solutions prepared according to some formulas, the yohimbine cannot be completely dissolved, and at this time, there may be some trace suspended substances or some undissolved crystal nuclei in the concentrated solutions. In order to obtain a stable yohimbine concentrated solution, it is necessary to select the number of operations of step 5 according to the situation of the prepared concentrated solution, thereby fully filtering the impurities in the prepared yohimbine concentrated solution.

Further, Example 5 of the present application further provides a portion of specific component formulas for preparing the foregoing yohimbine concentrated solution, referring to Table 2 below.

Table 2 Formulas of yohimbine concentrated solution

| | Yohimbine sodium carbonate | Benzyl alcohol | Water | Glacial acetic acid |
|---|---|---|---|---|
| 1 | 10% | 90% | / | / |
| 2 | 20% | 80% | / | / |
| 3 | 10% | / | 10% | 80% |
| 4 | 20% | / | 40% | 40% |
| 5 | 25% | / | 50% | 25% |
| 6 | 5% | 5% | 80% | 10% |
| 7 | 20% | 10% | 50% | 20% |
| 8 | 30% | 30% | 25% | 15% |
| 9 | 35% | 27% | 13% | 25% |
| 10 | 40% | 16% | 28% | 16% |

It should be noted that, Table 1 only lists a portion of formulas for preparing the yohimbine concentrated solution. Under the condition of meeting the mass content proportion of each component provided in the example of the present application, adaptive adjustment can be made according to the specific concentration need of the yohimbine extract.

Example 7 of the present application further performs comparative analysis of the solubility of atomized solutions by adding different yohimbine extracts to a solvent of a glycerol-propylene glycol system. A specific analysis method is as follows:

(1) Preparation of a liquid preparation a: The yohimbine hydrochloride was added to the solvent of the glycerol-propylene glycol system, based on the weight of the liquid preparation a, the mass ratio content of the yohimbine hydrochloride was 2%; and the liquid preparation a was heated for 30 min at a heating temperature of 60°C.

(2) Preparation of a liquid preparation b: The yohimbine obtained by the foregoing deacidification process in Example 2 was added to the solvent of the glycerol-propylene glycol system, based on the weight of the liquid preparation b, the mass ratio content of the yohimbine was 2%; and the liquid preparation b was heated for 30 min at a heating temperature of 60°C.

(3) Preparation of a liquid preparation c: The yohimbine concentrated solution provided in Example 5 of the present application was added to the solvent of the glycerol-propylene glycol system, based on the weight of the liquid preparation c, the mass percentage content of the yohimbine was 2%; and the liquid preparation c was heated for 30 min at a heating temperature of 60°C.

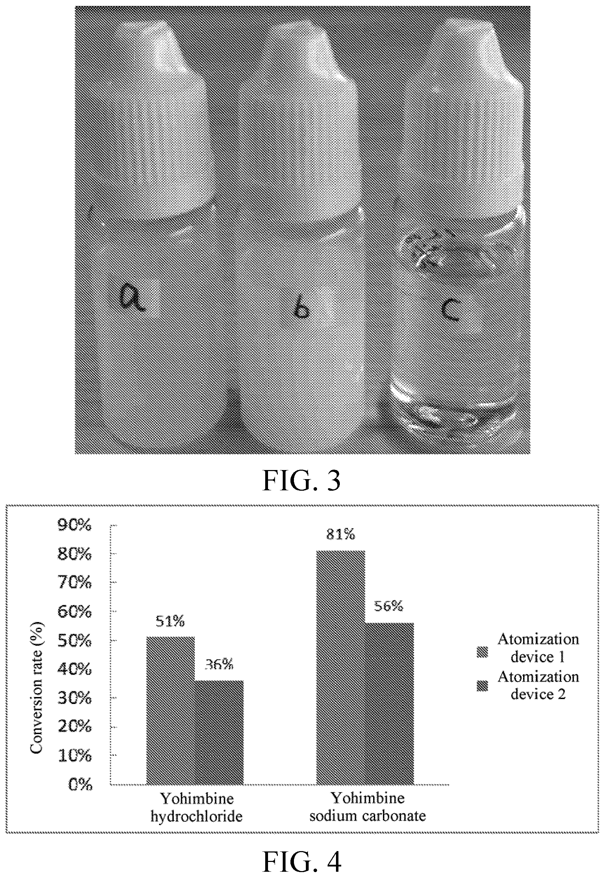

The dissolution situations of three groups of solutions, namely the liquid preparation a, the liquid preparation b and the liquid preparation c, were observed to obtain dissolved states of the three groups of solutions, as shown in Table 2 and FIG. 3.

Table 3 Dissolved states of different yohimbine raw materials

| Name | Liquid preparation a | Liquid preparation b | Liquid preparation c |
|---|---|---|---|
| Dissolved state | Insoluble | Insoluble | Transparent solution |

Referring to Table 3 and FIG. 3, the liquid preparation a and the liquid preparation b are turbid solutions, thus indicating that the solubility of the yohimbine hydrochloride in the solvent of the glycerol-propylene glycol system is very low, and the solubility of the yohimbine prepared by the deacidification process in the solvent of the glycerol-propylene glycol system is also very low; and the liquid preparation c is a clear and transparent solution, and after the liquid preparation c stands for a period of time, the solution c is still transparent with no crystal precipitation, thus indicating that the yohimbine concentrated solution provided in Example 5 of the present application can form a stable and transparent solution system in the solvent of the glycerol-propylene glycol system. The problem of adding the yohimbine to the liquid preparation can be solved by preparing the yohimbine into the yohimbine concentrated solution, the addition amount of the yohimbine extract can be increased according to specific use needs, and the prepared atomized liquid preparation has good stability and no precipitation.

Example 9 of the present application further performed comparative analysis of conversion rate effects of yohimbine in liquid preparations containing different yohimbine extracts converted to aerosols in the aerosol generation apparatus. Two groups of liquid preparations containing yohimbine extracts, namely a liquid preparation 3 and a liquid preparation 4, were prepared respectively, the liquid preparation 3 was atomized in the aerosol generation apparatus to form an aerosol 3, and the liquid preparation 4 was atomized in the aerosol generation apparatus to form an aerosol 4.

The liquid preparation 3 was prepared by adding the yohimbine hydrochloride to the solvent of the glycerol-propylene glycol system. Calculated by the weight percentage of the liquid preparation 3, the mass percentage of the yohimbine hydrochloride was 1%, the mass percentage of the propylene glycol was 49%, and the mass percentage of the glycerol was 50%.

The liquid preparation 4 was prepared by adding the yohimbine concentrated solution to the solvent of the glycerol-propylene glycol system. Calculated by the weight percentage of the liquid preparation 4, the mass percentage content of the yohimbine concentrated solution was 30%, the mass percentage of the propylene glycol was 20%, and the mass percentage of the glycerol was 50%. In the yohimbine concentrated solution used by the liquid preparation 4, calculated by the weight of the yohimbine concentrated solution, the mass percentage content of the yohimbine was 3.33%; and by calculation, it can be concluded that calculated by the weight of the liquid preparation 4, the mass percentage content of the yohimbine was 1%.

Example 10 of the present application provides an aerosol generation apparatus for an aerosol generation system. The aerosol generation apparatus includes an atomizer with an atomization function. The atomizer includes a heating element and a liquid guide element. The liquid guide element can absorb and transfer a liquid preparation, and the heating element can atomize the liquid preparation to form an aerosol. According to different materials used for the liquid guide element and different preparation processes of the atomizer, the atomizer includes a ceramic core atomizer and a cotton core atomizer. In the cotton core atomizer, the liquid guide element may be made of materials with a capillary structure and excellent liquid storage performance, such as non-woven fabrics and cotton; the heating element may be a spiral heating wire or heating mesh made of at least one of stainless steel, nickel chromium alloy, iron chromium aluminum alloy and metallic titanium; the liquid guide element may be fixedly arranged outside the spiral heating wire or heating mesh; or, the spiral heating wire or heating mesh is arranged around at least a portion of the surface of the liquid guide element. In the ceramic core atomizer, the liquid guide element may be a porous structure made of a hard capillary structure such as porous ceramics, porous glass ceramics or porous glass, the liquid guide element is substantially block-shaped, and the heating element is fixed on at least a portion of the surface of the liquid guide element; the heating element may be one of a heating coating, a heating sheet or a heating mesh, where the heating coating may include, but is not limited to, electromagnetic induction heating paint, infrared induction heating paint, and the like; or, conductive raw material powder can be mixed with printing auxiliaries to form slurry which is sintered on the surface of a porous body after printing to obtain the heating element. Example 9 of the present application further performs comparative analysis of the impact of the ceramic core atomizer and the cotton core atomizer on the yohimbine conversion rate in the liquid preparation.

Example 9 of the present application performs test analysis of the yohimbine conversion rate in the aerosol 3 and the aerosol 4. The used test method refers to the following description.

The test method includes the following steps: a suction test was respectively performed on the liquid preparation 3 and the liquid preparation 4 by an exhaust hood according to international standards. Specific suction test conditions are as follows: each suction time was 3 s, the suction time interval was 30 s, the number of times of suction was 50, and the dose of the liquid preparation in each suction was 55 mL. The aerosol 3 and the aerosol 4 were respectively collected by a Cambridge filter. The yohimbine hydrochloride in the aerosol 3 was extracted by an organic solvent, and filtration was performed to obtain the yohimbine hydrochloride. The yohimbine in the aerosol 4 was extracted by an organic solvent, and filtration was performed to obtain the yohimbine. The conversion rate of the yohimbine hydrochloride in the aerosol 3 formed by atomization of the yohimbine hydrochloride in the liquid preparation 3 and the conversion rate of the yohimbine in the aerosol 4 formed by atomization of the yohimbine in the liquid preparation 4 were respectively computed according to the following calculation formula of the conversion rate by a liquid chromatography analysis method.

$$\mathrm{w\%} = \frac{C_1 \times A_0}{A_1 \times C_0} \times 100\%$$

Calculation formula of conversion rate: where $C_0 = m_0/V$, $C_1 = m_1/V$.

In the foregoing calculation formula of the conversion rate: $W\%$ represents the conversion efficiency of the yohimbine; $C_0$ represents the concentration of the aerosol captured by the Cambridge filter; $C_1$ represents the concentration of the liquid preparation; $A_0$ represents the chromatographic peak area of the yohimbine in the aerosol; $A_1$ represents the chromatographic peak area of the yohimbine in the liquid preparation; $m_0$ represents the mass of the aerosol (namely the mass difference before and after capture by the Cambridge filter); $m_1$ represents the mass of the liquid preparation; and V represents the volume of an extraction solvent.

In Example 9 provided in the present application, the liquid preparation 3 was respectively added to an aerosol generation apparatus 1 of the same specification provided with a cotton core atomizer, and an aerosol generation apparatus 2 of the same specification provided with a ceramic core atomizer; and the liquid preparation 4 was respectively added to an aerosol generation apparatus 3 of the same specification provided with a cotton core atomizer, and an aerosol generation apparatus 4 of the same specification provided with a ceramic core atomizer. Then, the aerosol generation apparatus 1, the aerosol generation apparatus 2, the aerosol generation apparatus 3 and the aerosol generation apparatus 4 were respectively connected to the same exhaust hood to respectively obtain a first group of aerosols, a second group of aerosols, a third group of aerosols and a fourth group of aerosols according to the foregoing suction test method. Then, a same organic solvent was used for extracting a yohimbine hydrochloride sample 1 in the first group of aerosols, a same organic solvent was used for extracting a yohimbine hydrochloride sample 2 in the second group of aerosols, a same organic solvent was used for extracting a yohimbine sample 3 in the third group of aerosols, and a same organic solvent was used for extracting a yohimbine sample 4 in the fourth group of aerosols. Then, the sample 1, the sample 2, the sample 3 and the sample 4 were respectively added to a liquid chromatography analyzer to perform liquid chromatography analysis to obtain a chromatographic peak area $A_{01}$ of the yohimbine in the sample 1, a chromatographic peak area $A_{02}$ of the yohimbine in the sample 2, a chromatographic peak area $A_{03}$ of the yohimbine in the sample 3, and a chromatographic peak area $A_{04}$ of the yohimbine in the sample 4.

The liquid preparation 3 and the liquid preparation 4 were prepared, where the mass of the liquid preparation 3 was $m_{13}$, and the mass of the liquid preparation 4 was $m_{14}$. The liquid preparation 3 and the liquid preparation 4 were respectively added to a liquid chromatograph to perform liquid chromatography analysis to obtain a chromatographic peak area $A_{13}$ corresponding to the yohimbine in the liquid preparation 3, and a chromatographic peak area $A_{14}$ corresponding to the yohimbine in the liquid preparation 4.

Then, $A_{01}$ and $A_{13}$ were substituted into the foregoing calculation formula of the conversion rate to obtain conversion efficiency of the yohimbine hydrochloride in the first group of aerosols formed by atomization of the liquid preparation of the yohimbine hydrochloride in the aerosol generation apparatus 1 provided with the cotton core atomizer; $A_{02}$ and $A_{13}$ were substituted into the foregoing calculation formula of the conversion rate to obtain conversion efficiency of the yohimbine hydrochloride in the second group of aerosols formed by atomization of the liquid preparation of the yohimbine hydrochloride in the aerosol generation apparatus 2 provided with the ceramic core atomizer; $A_{03}$ and $A_{14}$ were substituted into the foregoing calculation formula of the conversion rate to obtain conversion efficiency of the yohimbine in the third group of aerosols formed by atomization of the liquid preparation of the yohimbine concentrated solution in the aerosol generation apparatus 3 provided with the cotton core atomizer; and $A_{04}$ and $A_{14}$ were substituted into the foregoing calculation formula of the conversion rate to obtain conversion efficiency of the yohimbine in the fourth group of aerosols formed by atomization of the liquid preparation of the yohimbine concentrated solution in the aerosol generation apparatus 4 provided with the ceramic core atomizer.

Finally, the results of the four groups of comparative analysis tests are shown in FIG. 4. From the figure, it can be seen that in the aerosol generation apparatus provided with the ceramic core, corresponding to the atomization apparatus 1 in FIG. 4, in the aerosol formed by atomization of the liquid preparation of the yohimbine hydrochloride, the conversion rate of the yohimbine is 51%, and in the aerosol formed by atomization of the liquid preparation of the yohimbine concentrated solution, the conversion rate of the yohimbine is 81%, that is, the liquid preparation prepared by the yohimbine concentration process provided in the examples of the present application can significantly increase the content of the yohimbine in the aerosol. In the aerosol generation apparatus provided with the cotton core, corresponding to the atomization apparatus 2 in FIG. 4, in the aerosol formed by atomization of the liquid preparation of the yohimbine hydrochloride, the conversion rate of the yohimbine is 36%, and in the aerosol formed by atomization of the liquid preparation of the yohimbine concentrated solution, the conversion rate of the yohimbine is 56%, that is, the liquid preparation prepared by the yohimbine concentration process provided in the examples of the present application can significantly increase the content of the yohimbine in the aerosol. In the same liquid preparation containing yohimbine, the content of the yohimbine in the aerosol formed by atomization using the aerosol generation apparatus provided with the ceramic core atomizer is N1, and the content of the yohimbine in the aerosol formed by atomization using the aerosol generation apparatus provided with the cotton core atomizer is N2, where N1>N2. Thus, the aerosol generation apparatus provided with the ceramic core atomizer has a better atomization effect on the liquid preparation containing yohimbine, and can increase the conversion rate of the yohimbine.

Example 11 of the present application further provides a liquid preparation containing yohimbine for an aerosol generation system. The liquid preparation includes a yohimbine concentrated solution, a solvent, a sweetening agent, a cooling agent and a flavor composition. Based on the weight of the liquid preparation, the mass percentage content of the yohimbine concentrated solution is 0.1-30%; the mass percentage content of the solvent is 50-99.9%; the mass percentage content of the sweetening agent is 0.01-0.5%; the mass percentage content of the cooling agent is 0.01-8%; and the mass percentage content of the flavor composition is 0.01-15%.

In the liquid preparation provided in Example 11 of the present application, the solvent includes, but is not limited to, one or a mixture of several of propylene glycol, glycerol, butanediol, 1,2,4-butantriol, dipropylene glycol, diethylene glycol, triethylene glycol, polyethylene glycol 200, polyethylene glycol 400, glycerol triacetate, dipropylene glycol ether, ethanol, water, triethyl citrate and caprylic/capric triglyceride.

In the liquid preparation provided in Example 11 of the present application, the cooling agent includes, but is not limited to, one or a combination of several of menthol, menthone, isomenthone, WS-23(2-isopropyl-N,2,3-trimethylbutanamide), WS-3(N-ethyl-p-menthyl-3-formamide), WS-5(N-(ethoxycarbonylmethyl)-p-alkyl-3-formamide), menthyl lactate and menthone glycerol acetal, and the total addition amount is 0.01%-8% (based on the mass percentage content).

In the liquid preparation provided in Example 11 of the present application, the sweetening agent includes, but is not limited to, one or a combination of several of neotame, sodium cyclamate, sucralose, aspartame, stevioside and acesulfame, and the total addition amount is 0.01-0.5% (based on the mass percentage content).

In the liquid preparation provided in Example 11 of the present application, the flavor composition is a natural or synthetic essence, including but not limited to flavors such as fruits, floral aroma, tea aroma, mint, coffee, soda, candy, or tobacco. Adding the flavor composition can enrich the taste of the aerosol formed by atomization of the liquid preparation.

Example 12 of the present application further provides an aerosol generation apparatus. The aerosol generation apparatus is provided with a cartridge capable of containing the liquid preparation provided in any example of the present application, and the liquid preparation contains a yohimbine extract. An atomizer capable of atomizing the liquid preparation to form an aerosol and a control module capable of stably releasing the aerosol are arranged inside the aerosol generation apparatus. The atomizer can be driven by a power source for atomization, and the aerosol is outputted to the outside of the aerosol generation apparatus through an aerosol output channel inside the aerosol generation apparatus for a user to inhale. In addition to electrical driven atomization, the aerosol generation apparatus can also atomize the liquid preparation to form an aerosol through other forms, so that the aerosol is effectively transferred to the user. The specific atomization form can be optimized and selected according to the atomization temperature of specific components of a liquid substrate inside the cartridge of the aerosol generation apparatus without limitation. In some embodiments, the aerosol generation apparatus can perform atomization by a resistor and/or an electromagnetic heating body. In other embodiments, ultrasonic atomization can be performed, or electric heating atomization and ultrasonic atomization can be performed simultaneously. In some embodiments, the aerosol generation apparatus may also be an air compression type atomization apparatus or a press type spraying apparatus.

It should be noted that, the specification and accompanying drawings of the present application provide preferred examples of the present application, but are not limited to the examples described in the specification. Further, a person of ordinary skill in the art may make improvements or modifications according to the foregoing descriptions, and all the improvements and modifications shall fall within the protection scope of the appended claims of the present application.

**Claims**

1.  A liquid preparation for an aerosol generation system, wherein the liquid preparation comprises a yohimbine extract, a chaotropic agent and a solvent,
    wherein the solubility of the yohimbine extract in the chaotropic agent is greater than the solubility of the yohimbine extract in the solvent, and the yohimbine extract can form a stable and transparent solution with the solvent after being dissolved in the chaotropic agent.

2. The liquid preparation according to claim 1, wherein based on the mass percentage of the liquid preparation, the mass percentage content of the yohimbine extract is 0.01-15%, the mass percentage content of the chaotropic agent is 0.09-15%, and the mass percentage content of the solvent is 50-99.9%.

3. The liquid preparation according to claim 1 or 2, wherein the yohimbine extract comprises Yu-7-binine.

4. The liquid preparation according to claim 1, wherein the yohimbine and the chaotropic agent form a yohimbine concentrated solution; and
based on the weight percentage of the liquid preparation, the mass percentage content of the yohimbine concentrated solution is 0.1-30%.

5. The liquid preparation according to claim 1 or 2, wherein the solvent comprises glycerol and propylene glycol.

6. The liquid preparation according to claim 3, wherein based on the weight percentage of the liquid preparation, the mass percentage content of the yohimbine extract is 0.015-12%, or the mass percentage content of the yohimbine extract is 0.02-12%.

7. The liquid preparation according to claim 1 or 2, wherein the chaotropic agent comprises at least one of benzyl alcohol and organic acid.

8. The liquid preparation according to claim 7, wherein the chaotropic agent further comprises water.

9. The liquid preparation according to claim 7, wherein the organic acid comprises at least one of lactic acid, glacial acetic acid, adipic acid, fumaric acid, tartaric acid, malic acid, citric acid and benzoic acid.

10. The liquid preparation according to claim 7, wherein based on the weight percentage of the liquid preparation, the mass percentage content of the benzyl alcohol is 0-27%;

   or, the mass percentage content of the benzyl alcohol is 0.008-15%;
   or, the mass percentage content of the benzyl alcohol is 0.012-9%.

11. The liquid preparation according to claim 7, wherein based on the weight percentage of the liquid preparation, the mass ratio content of the organic acid is 0-24%;

   or, the mass percentage content of the organic acid is 0.008-15%;
   or, the mass percentage content of the organic acid is 0.012-9%.

12. The liquid preparation according to claim 8, wherein based on the weight percentage of the liquid preparation, the mass percentage content of the water is 0-24%;

   or, the mass percentage content of the water is 0.01-15%;
   or, the mass percentage content of the water is 0.02-12%.

13. The liquid preparation according to claim 1, wherein the liquid preparation further comprises at least one of a sweetening agent, a cooling agent and a flavor composition.

14. A method for preparing the liquid preparation according to any one of claims 1 to 13, comprising the following steps:

   preparing yohimbine;
   adding the yohimbine to a chaotropic agent to enable the yohimbine to be fully dissolved in the chaotropic agent to obtain a yohimbine concentrated solution; and
   adding the yohimbine concentrated solution to a solvent.

15. The method for preparing the liquid preparation according to claim 14, wherein the yohimbine is prepared from yohimbine hydrochloride by a deacidification process.

**16.** A cartridge for an aerosol generation system, wherein the cartridge contains a liquid preparation, and the liquid preparation comprises the liquid preparation according to any one of claims 1 to 13.

**17.** An aerosol generation system, wherein the aerosol generation system comprises the cartridge according to claim 16 and an aerosol generation apparatus; and

the aerosol generation apparatus is constructed to receive at least a portion of the cartridge and atomize the liquid preparation to form an aerosol.

**18.** An aerosol generation apparatus, wherein the aerosol generation apparatus comprises an atomizer and a chamber containing the liquid preparation according to any one of claims 1 to 13, and the atomizer is configured to atomize the liquid preparation to generate an aerosol.

FIG. 1

FIG. 2

FIG. 3

FIG. 4

### INTERNATIONAL SEARCH REPORT

| International application No. |
| --- |
| **PCT/CN2022/126035** |

**A.    CLASSIFICATION OF SUBJECT MATTER**

A61K 9/12(2006.01)i；  A61K 31/475(2006.01)i；  A61M 11/00(2006.01)i

According to International Patent Classification (IPC) or to both national classification and IPC

**B.    FIELDS SEARCHED**

Minimum documentation searched (classification system followed by classification symbols)

A61K; A61M

Documentation searched other than minimum documentation to the extent that such documents are included in the fields searched

Electronic data base consulted during the international search (name of data base and, where practicable, search terms used)

WPABSC, CNTXT, CJFD, ENTXTC: 育亨宾, 可立宁, 萎必治, 安慰乐得, 气溶胶, 气胶, 雾化, 喷雾, 溶剂, 甘油, 丙三醇, 丙二醇, 苯甲醇, 酸, 合元科技; WPABS, ENTXT, VEN: yohimbine, corynine, aphrodyne, antagonil, aerosol, pulverization, atomization, solvent, glycerol, glycerin, propylene glycol

**C.    DOCUMENTS CONSIDERED TO BE RELEVANT**

| Category* | Citation of document, with indication, where appropriate, of the relevant passages | Relevant to claim No. |
| --- | --- | --- |
| PY | CN 115191639 A (SHENZHEN FIRST UNION TECHNOLOGY CO., LTD.) 18 October 2022 (2022-10-18) <br> claims 7 and 10, and description, paragraph [0019] | 1-18 |
| Y | WO 2011074015 A2 (THEMIS MEDICARE LTD.) 23 June 2011 (2011-06-23) <br> description, page 8, paragraph 2, page 9, paragraph 4, page 17, paragraph 4, page 18, paragraph 5, page 19, paragraph 2, page 21, paragraphs 2-3, and embodiments 1, 2, and 8 | 1-18 |
| Y | US 2016143974 A1 (CHONG CORP.) 26 May 2016 (2016-05-26) <br> claims 1, 42, and 44 | 1-18 |
| Y | CN 102030747 A (SHAANXI JIAHE PHYTOCHEM CO., LTD.) 27 April 2011 (2011-04-27) <br> claim 2 | 15 |
| A | CN 1700934 A (CHRYSALIS TECHNOLOGIES INC.) 23 November 2005 (2005-11-23) <br> claims 1-30 | 1-18 |
| A | GB 190011647 A (SPIEGEL LEOPOLD) 11 August 1900 (1900-08-11) <br> claims 1-4 | 15 |

☐ Further documents are listed in the continuation of Box C.      ☑ See patent family annex.

| \* | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 January 2023** | **18 January 2023** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
|---|
| **PCT/CN2022/126035** |

| Patent document cited in search report | | | Publication date (day/month/year) | Patent family member(s) | | | Publication date (day/month/year) |
|---|---|---|---|---|---|---|---|
| CN | 115191639 | A | 18 October 2022 | None | | | |
| WO | 2011074015 | A2 | 23 June 2011 | WO | 2011074015 | A3 | 26 April 2012 |
| US | 2016143974 | A1 | 26 May 2016 | US | 10098918 | B2 | 16 October 2018 |
| CN | 102030747 | A | 27 April 2011 | None | | | |
| CN | 1700934 | A | 23 November 2005 | US | 2004081624 | A1 | 29 April 2004 |
| | | | | AU | 2003263061 | A1 | 29 March 2004 |
| | | | | CA | 2497845 | A1 | 18 March 2004 |
| | | | | EP | 1539284 | A2 | 15 June 2005 |
| | | | | HK | 1085952 | A1 | 08 September 2006 |
| | | | | WO | 2004022128 | A2 | 18 March 2004 |
| | | | | JP | 2005538159 | A | 15 December 2005 |
| GB | 190011647 | A | 11 August 1900 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111248947